# FASCICULE DE BREVET EUROPEEN

(11) **EP 1 932 818 B1**
(45) Date de publication et mention de la délivrance du brevet: **25.06.2014**
(21) Numéro de dépôt: 07291404.7
(22) Date de dépôt: 23.11.2007
(51) Int. Cl.: C07B 63/00, C07C 41/06, C07C 43/04, B01D 11/04, C07C 7/10

(54) **Elimination de l'acétonitrile dans la charge oléfinique des procédés de production d'éthers par mise en oeuvre de liquides ioniques**
Entfernung von Acetonitril aus einem im Herstellungsverfahren von Ethern zu verwendenden olefinischen Einsatzstoffes unter Verwendung von ionischen Flüssigkeiten
Ionic liquid mediated elimination of acetonitrile from the olefin feed used in the production of ethers.

(30) Priorité: 12.12.2006 FR 0610974
(43) Date de publication de la demande: 18.06.2008
(73) Titulaire: IFP Energies nouvelles, 92852 Rueil-Malmaison Cedex (FR)
(72) Inventeur: Cadours, Renaud, 69340 Francheville (FR); Forestiere, Alain, 69390 Vernaison (FR); Vallee, Christophe, 38600 Fontaine (FR)

(56) Documents cités:
- WO-A1-2005/019137
- FR-A1- 2 875 235
- US-A- 5 120 881
- US-A- 5 672 772
- US-A- 5 684 212
- US-A1- 2005 010 076

## Description

### DOMAINE DE L'INVENTION

L'invention concerne un procédé d'élimination de l'acétonitrile présent dans les coupes oléfiniques contenant au moins une iso-oléfine alimentant les procédés de production d'éthers par mise en oeuvre de liquides ioniques.

### ART ANTÉRIEUR

Les procédés de production d'éther carburant consistent traditionnellement à additionner un alcool sur une oléfine ramifiée. On peut citer le procédé de fabrication du méthyl tertiobutyl éther (MTBE) dans lequel on additionne du méthanol sur de l'isobutène, contenu par exemple dans une coupe C4 de vapocraquage ou de craquage catalytique ou de déshydrogénation de l'isobutane. Un procédé analogue permet de produire l'éthyl tertiobutyl éther (ETBE) à partir d'éthanol et d'isobutène, mais également différents éthers tels que l'isopropyl tertiobutyl éther (IPTBE) à partir d'isopropanol et d'isobutène, le tertio amyl méthyl ether (TAME) à partir de méthanol et d'isoamylène, issus de coupes C5 ex FCC ou ex vapocraqueur ou du procédé Isofive d'isomérisation équilibrée des n-pentènes, ou encore l'éthyl tertioamyl éther (ETAE) à partir d'éthanol et d'isoamylène.

D'une manière générale, les procédés industriels comprennent une section réactionnelle dans laquelle l'éther est produit en phase liquide, à basse température, par exemple 50 °C, par réaction d'une coupe oléfinique contenant au moins une iso-oléfine sur un monoalcool, en présence d'un catalyseur de type résine échangeuse d'ions, dans un ou plusieurs réacteurs en série.

La réaction d'éthérification est très sélective vis-à-vis des iso-oléfines de la coupe C4 mais est réalisée avec un excès d'alcool de façon à déplacer l'équilibre de la réaction vers la production d'éther. La composition de la coupe C4 varie selon son origine, celle-ci pouvant être le vapocraquage, le craquage catalytique, mais aussi la déshydrogénation d'isobutane ou encore la déshydratation de tertiobutanol. Elle contient généralement moins de 50 % poids d'iso-oléfines, le reste de la charge étant composé d'un mélange d'hydrocarbures inertes vis-à-vis de la réaction d'éthérification.

L'étape réactionnelle d'éthérification est ensuite suivie d'une étape de séparation qui a pour objectif de séparer la fraction éther formée et les hydrocarbures non réactifs ou n'ayant pas réagi en vue de leur utilisation ultérieure, et l'alcool excédentaire. Cette section de séparation peut être constituée d'une colonne de fractionnement permettant de récupérer en fond l'éther et la coupe hydrocarbure en tête de colonne. L'alcool non converti est récupéré, principalement à partir de la coupe hydrocarbure, et recyclé en amont de la section réactionnelle.

La réaction principale entre l'oléfine et l'alcool est concurrencée par une réaction parallèle d'addition d'eau sur l'oléfine. Par exemple, dans le cas de la production d'ETBE à partir d'isobutène et d'éthanol, cette réaction parallèle conduit à la formation de tertiobutyl alcool (TBA). Il est donc souhaitable de minimiser la teneur en eau dans l'alimentation du réacteur d'éthérification.

L'eau a trois principales origines dans le procédé, correspondant aux teneurs résiduelles en eau de l'alcool et de la charge oléfinique utilisée, et à la teneur en eau de l'alcool recyclé. En effet, ce dernier est généralement extrait par un lavage à l'eau de la fraction hydrocarbonée obtenue en sortie de la section de séparation. L'alcool recyclé contient alors une forte teneur en eau. Ce recyclage peut apporter jusqu'à 50 % de l'eau alimentant la section réactionnelle. La demande de brevet FR-A-2 900 924 déposée au nom de la Demanderesse propose une alternative au lavage à l'eau de la coupe hydrocarbonée pour la récupération de l'alcool. Cette invention réduit alors le problème de l'introduction d'eau dans le procédé de production d'éthers aux alimentations du procédé : l'alcool et la charge oléfinique.

La teneur en eau de la coupe oléfinique est liée aux opérations de traitement de cette coupe, en amont du procédé d'éthérification. En effet, la coupe oléfinique présente des teneurs variables en acétonitrile, selon qu'elle est issue du vapocraquage, du FCC, d'une opération de déshydrogénation ou de déshydratation. Cette teneur est inférieure à 20 ppm, La présence de cette impureté dans la charge oléfinique représente un risque important d'inhibition du catalyseur utilisé, à savoir des résines échangeuses d'ions.

On observe une association irréversible entre l'acétonitrile et les catalyseurs utilisés dans les réacteurs d'éthérification. Il est donc indispensable de procéder à des opérations d'élimination de l'acétonitrile présent avant de pouvoir envoyer la charge oléfinique dans la section réactionnelle.

Généralement, l'élimination de cette impureté est réalisée par lavage à l'eau dans une section dédiée à ce traitement. L'inconvénient de la solution est alors la saturation en eau de la charge oléfinique. La quantité d'eau introduite par ce lavage représente la moitié au moins de l'eau qui entre dans le procédé.

En général, toute molécule de nitrile qui pénètre dans la section réactionnelle d'éthérification et qui a donc échappé à la capture par l'unité de lavage de la charge est piégée par les résines catalyseur (qui perdent ainsi leur caractère acide et donc leur activité catalytique) soit dans une première passe soit encore parce que ces nitriles sont recyclés au travers du recyclage de l'alcool comme indiqué dans le brevet US 5 352 848 .

La mise en oeuvre d'un procédé utilisant un liquide d'extraction différent de l'eau pour l'extraction de l'acétonitrile permet de diviser en général au moins par deux la quantité d'eau à l'entrée du réacteur d'éthérification.

### RÉSUMÉ DE L'INVENTION

La présente invention présente un procédé amélioré de fabrication d'éthers à partir d'une coupe oléfinique contenant au moins une iso-oléfine et d'un alcool comprenant, avant éthérification, une étape d'élimination de l'acétonitrile présent dans la charge hydrocarbonée par extraction liquide-liquide, réalisée dans au moins une zone d'extraction, le solvant d'extraction étant un liquide ionique non aqueux de formule générale Q⁺A⁻, dans laquelle Q⁺ est un cation ammonium-et A⁻ un anion formant avec ledit cation un sel liquide.

Avantageusement, le procédé selon l'invention permet de diviser au moins par deux la quantité d'eau à l'entrée du réacteur d'éthérification et ainsi de limiter la désactivation du catalyseur.

### DESCRIPTION DES DESSINS

La Figure 1 représente un schéma du procédé d'élimination de l'acétonitrile présent dans la charge hydrocarbonée par mise en oeuvre de liquides ioniques.
La Figure 2 représente un schéma du procédé de production d'ETBE à partir d'isobutène et d'éthanol, comprenant un recyclage d'une fraction de l'effluent sortant du réacteur d'éthérification.

### DESCRIPTION DÉTAILLÉE DE L'INVENTION

L'invention propose un procédé de production d'éthers comportant avant éthérification une étape d'élimination de l'acétonitrile présent dans une charge oléfinique contenant au moins une iso-oléfine destinée à la fabrication d'éther par extraction liquide-liquide avec, comme solvant, un liquide ionique non-aqueux de formule générale Q⁺A⁻, dans laquelle le cation Q⁺ dérivé d'hétérocycle azoté comportant 1 ou 2 d'azote, l'hétérocycle étant constitué de 5 à 6 atomes, de formules générales 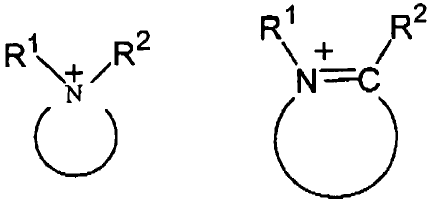
dans lesquelles R¹ et R², identiques ou différents, représentent l'hydrogène, de préférence un seul substituant représentant l'hydrogène, ou des radicaux hydrocarbyles ayant de 1 à 30 atomes de carbone
et A- un anion formant avec ledit cation un sel liquide.

Cette étape d'extraction a lieu préalablement à l'étape réactionnelle d'éthérification réalisée en présence d'une résine échangeuse d'ions. Elle permet donc de diminuer de façon très avantageuse la quantité d'eau entrant dans le réacteur d'éthérification et par conséquence de limiter la réaction d'hydrolyse de l'oléfine.

La charge hydrocarbonée comprend moins de 50% poids d'iso-oléfines destinées à la fabrication d'éthers.

Pour réaliser cette extraction, le liquide ionique choisi comme solvant est ajouté à la charge hydrocarbonée contenant environ de 10 à 20 ppm d'acétonitrile et l'ensemble est introduit dans une colonne d'extraction liquide-liquide.

Par leur nature, les liquides ioniques ne présentent pas de coefficient de partage avec la charge hydrocarbonée, ces deux entités étant non-miscibles. Il n'y a par conséquent pas lieu de considérer les pertes en liquide ionique par partage avec la charge à traiter. Le risque d'entraînement mécanique n'est pas non plus limitant en raison de la large différence de densité entre les deux phases.

Ces techniques peuvent consister en un contact à co-courant ou à contre-courant des deux phases liquides. On peut utiliser un ou plusieurs mélangeurs-décanteurs ou une colonne de contact liquide-liquide. Dans ce cas, on peut mettre en oeuvre par exemple une colonne à disques, une colonne à plateaux, une colonne pulsée, une colonne à garnissage (vrac ou structuré). Pour réaliser le contact entre les phases, on peut encore envisager d'utiliser un contacteur membranaire. Ces différentes techniques peuvent être utilisées seules ou en association. On utilisera de préférence des techniques à contre-courant. d'utiliser un contacteur membranaire. Ces différentes techniques peuvent être utilisées seules ou en association. On utilisera de préférence des techniques à contre-courant.

Le choix de la technique de séparation la plus adaptée se fera selon les règles déjà connues de l'homme du métier.

Le liquide ionique non-aqueux décrit dans cette invention est choisi dans le groupe formé par les sels liquides qui ont pour formule générale Q⁺A⁻ dans laquelle Q⁺ représente un cation tel que défini précedemment et A⁻ représente tout anion, organique ou inorganique, susceptible de former un sel liquide à basse température, c'est-à-dire en dessous de 100 °C et de préférence en dessous de 50°C.

Dans le liquide ionique non-aqueux de formule Q⁺A⁻, les anions A⁻ sont de préférence choisis parmi les anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroethyl)phosphate, hexafluoroantimonate, fluorosulfonate, alkylsulfonates (par exemple le methylsulfonate), perfluoroalkylsulfonates (par exemple le trifluorométhylsulfonate), bis(perfluoroalkylsulfonyl)amidures (par exemple l'amidure de bis trifluorométhylsulfonyle de formule N(CF₃SO₂)₂⁻), le méthylure de tris-trifluorométhylsulfonyle de formule C(CF₃SO₂)₃⁻, le méthylure de bis-trifluorométhylsulfonyle de formule HC(CF₃SO₃)₃⁻, arènesulfonates, éventuellement substitués par des groupements halogènes ou halogénoalkyles, l'anion tétraphénylborate et les anions tétraphénylborates dont les noyaux aromatiques sont substitués, le tétra-(trifluoroacétoxy)-borate, le bis-(oxalato)-borate, le dicyanamide et le tricyanométhylure.

Dans les formules, R¹, R², représentent l'hydrogène, de préférence un seul substituant représentant l'hydrogène, ou des radicaux hydrocarbyles ayant de 1 à 30 atomes de carbone, par exemple des groupements alkyles, saturés ou non saturés, cycloalkyles ou aromatiques, aryles ou aralkyles, éventuellement substitués, comprenant de 1 à 30 atomes de carbone.

R¹, R², peuvent également représenter des radicaux hydrocarbyles portant une ou plusieurs fonctions choisies parmi les fonctions -CO₂R. -C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

De manière préférée, le cation Q⁺ est choisi dans le groupe formé par le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazolium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium, le N-butyl-N-méthylpyrrolidinium.

À titre d'exemples de sels utilisables selon l'invention, on peut citer l'hexafluorophosphate de N-butylpyridinium, le bis(trifluorométhylsulfonyl) amidure de butyl-3-méthyl-1-imidazolium, le bis(trifluorométhylsulfonyl) amidure de butyl-3-diméthyl-1,2-imidazolium, le bis(trifluorométhylsulfonyl) amidure de N-butyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le bis(trifluorométhylsulfonyl)amidure de (hydroxy-2-éthyl)-1-méthyl-3-imidazolium, le bis(trifluorométhylsulfonyl)amidure de (carboxy-2-éthyl)-1-méthyl-3-imidazolium. Ces sels peuvent être utilisés seuls ou en mélange.

La description qui suit considère de façon non-limitative une colonne de séparation à contre-courant. Dans ce cas, la charge hydrocarbonée chargée en acétonitrile est introduite en bas de la colonne et est entraînée vers le haut. Elle se trouve ainsi mise en contact avec le liquide ionique introduit en haut de la colonne. De cette façon, le liquide ionique se charge en acétonitrile et la charge hydrocarbonée s'en trouve appauvrie.

Le mélange soutiré de la section d'extraction, en fond de colonne, est constitué du liquide ionique et de l'acétonitrile. Il est envoyé vers une section de régénération du liquide ionique, par exemple par distillation et/ou détente. Les différences entre les points d'ébullition des liquides ioniques et de l'acétonitrile permettent une séparation aisée des deux composés.

Avantageusement, le liquide ionique est ensuite recyclé pour l'extraction.

La charge hydrocarbonée purifiée extraite de la zone d'extraction et contenant moins de 0,5 ppm et de préférence moins de 0,1 ppm d'acétonitrile peut alors être envoyée vers la section réactionnelle, sans risque d'endommager les résines échangeuses d'ions.

La diminution de la teneur en eau permettant de limiter la réaction parallèle conduisant par exemple à la formation de tertiobutyl alcool (TBA) dans le cas de la production d'ETBE, l'activité catalytique de la résine peut être ainsi augmenter d'au moins 20 %. Elle permet également de diminuer la quantité de résine à mettre en oeuvre ou de travailler à plus basse température pour un même taux de conversion dans le cas d'une unité existante.

Le procédé de production d'éthers à partir d'une charge oléfinique contenant au moins une iso-oléfine et d'alcools selon l'invention comportent les étapes suivantes:
a) élimination de l'acétonitrile présent dans la fraction hydrocarbonée selon le procédé décrit précédemment,
b) mélange de la fraction hydrocarbonée obtenue après l'étape a) avec au moins un flux d'alcool,
c) réaction d'éthérification par réaction du mélange obtenu à l'étape b) en présence d'une résine échangeuse d'ions de manière à obtenir un effluent éther-hydrocarbures-alcool,
d) séparation dans une colonne de fractionnement dudit effluent éther-hydrocarbures-alcool en un premier effluent enrichi en éther et contenant une partie de l'alcool excédentaire et en un deuxième effluent enrichi en hydrocarbures contenant l'autre partie de l'alcool excédentaire.

La fraction hydrocarbonée contient moins de 50% poids d'iso-oléfines.

L'alcool introduit peut être du méthanol ou de l'éthanol.

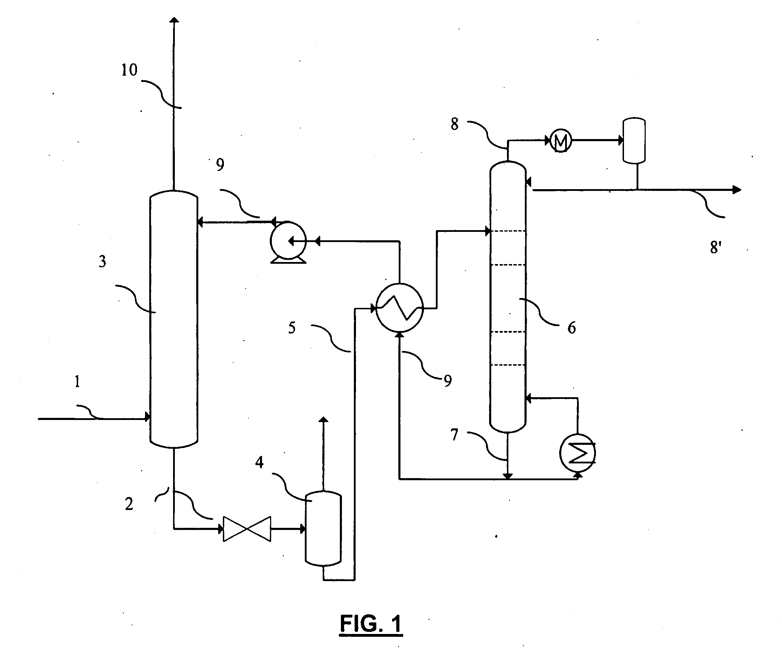

La présente invention va être décrite au regard de la Figure 1 représentant le procédé d'élimination d'acétonitrile par mise en oeuvre de liquides ioniques (sur cette figure seuls les équipements principaux sont détaillés).

De façon non-limitative, la Figure 1 considère une colonne d'extraction liquide-liquide fonctionnant à contre-courant, en considérant de façon non-limitative l'emploi d'un liquide ionique ayant une masse volumique supérieure à celle de la charge hydrocarbonée.

La fraction hydrocarbonée contenant des oléfines et de l'acétonitrile est acheminée au travers de la conduite (1) vers la colonne de séparation liquide-liquide (3). Le liquide ionique est introduit en haut de la colonne par la conduite (9). La circulation dans la colonne a lieu à contre-courant et l'effluent sortant de la colonne par la conduite (2) est constitué de liquide ionique chargé en acétonitrile.

Les conditions opératoires dans la section d'extraction sont généralement les conditions de disponibilité de la charge hydrocarbonée, à savoir une pression de 0,1 à 2 MPa et une température de 30 à 70 °C.

Si nécessaire, une première détente pour relarguer les espèces éventuellement co-absorbées dans le liquide ionique peut être réalisée dans le ballon de purge (4). Le liquide ionique chargé en acétonitrile est ensuite envoyé par la conduite (5) vers une colonne de distillation ou un évaporateur (6). Les conditions opératoires sont déterminées de façon à évaporer l'acétonitrile. On réalise en général cette opération à des pressions de 0,1 à 2 MPa, de préférence de 0,1 à 1 MPa.

Le fond de la colonne (6) constitué du liquide ionique est soutiré par la conduite (7). La conduite (9) permet de renvoyer le liquide ionique vers la colonne de séparation liquide-liquide (3), assurant ainsi son recyclage. En tête de colonne, on récupère l'acétonitrile via la conduite (8) qui sera ensuite envoyé vers une unité de traitement ou de valorisation par la conduite (8').

La charge hydrocarbonée contenant moins de 0,5 ppm et de préférence moins de 0,1 ppm d'acétonitrile est extraite de la colonne de séparation (3) par la conduite (10) et peut être envoyée dans la section réactionnelle du procédé d'éthérification.

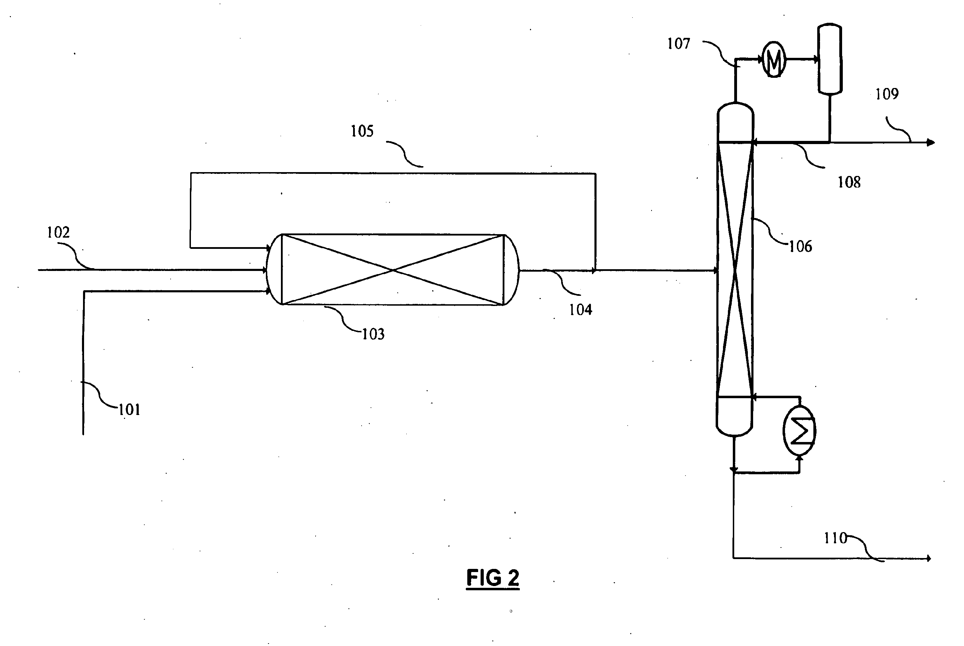

La Figure 2 représente un schéma du procédé d'éthérification. Une fraction hydrocarbonée contenant moins de 50 % en poids d'iso-oléfine et ayant été traitée dans l'unité d'élimination de l'acétonitrile est introduite dans le réacteur d'éthérification (103) par la conduite (101). Cette charge hydrocarbonée est ensuite mélangée avec la source externe d'alcool amenée par la conduite (102) et avec la fraction (105) de l'effluent sortant du réacteur qui est recyclée. L'effluent sortant de la section réactionnelle par la conduite (104) comprend un mélange d'hydrocarbures n'ayant pas réagi ou non réactifs, d'éther (produit de la réaction) et d'alcool non réagi. En général, cet effluent est composé de 10 à 60 % d'éther, de 1 à 10 % d'alcool et de 30 à 80 % d'hydrocarbures (rapports molaires), sa pression est de 0,8 à 2MPa et sa température de 50 °C à 90 °C.

L'effluent liquide issu du réacteur d'éthérification entre ensuite dans une colonne (106) de séparation, en vue de récupérer d'une part la fraction éther et d'autre part le mélange hydrocarbures-alcool. La colonne (106) peut être une colonne catalytique ou non, selon que la fraction introduite est plus ou moins riche en alcool.

En tête de la colonne (106), une conduite (107) amène un mélange gazeux hydrocarbures vers un échangeur puis vers un ballon de reflux qui permet de récupérer les vapeurs condensées par la conduite (108) et d'évacuer la fraction gazeuse par la conduite (109). Le mélange soutiré en fond de colonne (106) par la conduite (110) comprend essentiellement la phase éther et de l'alcool.

Les exemples suivants illustrent l'invention sans aucun caractère limitatif.

### EXEMPLES

### Exemple 1 selon l'invention

Différents essais de laboratoire réalisés en batch ont permis de montrer l'efficacité de la solution alternative proposée dans le cadre de la présente invention utilisant un liquide ionique à la place de l'eau pour traiter la charge hydrocarbonée en amont du procédé de production d'éther.

Le mode opératoire des essais réalisés est le suivant : 2 mL de solvant d'extraction (liquide ionique ou eau) et 6 mL de charge (une solution d'acétonitrile à 1000 ppm en poids dans l'heptane) sont agités pendant 30 minutes. Après 30 minutes de décantation, 2 g de la phase supérieure sont prélevés et 200 mg d'une solution étalon (solution de toluène à 0,2 % dans l'heptane) sont ajoutés, et le mélange est analysé par chromatographie en phase gazeuse. Différents liquides ioniques ont été testés et comparés à l'eau. Les résultats obtenus sont donnés dans le tableau suivant :

| Solvant d'extraction | Teneur résiduelle en acétonitrile dans l'hydrocarbure (ppm poids) après une seule étape de partage |
|---|---|
| [BMI][CF₃SO_{3]} (41 ppm d'eau). | 42 ppm |
| [BMI][PF₆] (85 ppm d'eau). | 28 ppm |
| [BMI][PF₆] (30 ppm d'eau). | 37 ppm |
| [BMPyrr][NTf2] (20 ppm d'eau) | 82 ppm |
| [BMI][CF₃CO_{2]} | 55 ppm |
| H₂O | 46 ppm |

Les liquides ioniques testés permettent une élimination au moins aussi efficace que l'eau généralement utilisée. Leur utilisation présente en outre l'avantage de diminuer fortement la teneur en eau dans l'alimentation de la section réactionnelle, par suppression de l'eau présente dans la coupe hydrocarbonée. Ainsi, même pour un liquide ionique présentant une efficacité moindre que celle de l'eau, la suppression d'introduction d'eau pour l'extraction de l'acétonitrile apporte un avantage important notamment par rapport à la quantité de sous-produits formés.

### Exemple 2 (comparatif)

En partant du schéma de base d'une unité de production d'ETBE tel que représenté à la Figure 2, une charge hydrocarbonée en C4 contenant 17 % molaire d'isobutène ayant une teneur en eau de 1500 ppm molaire est introduite dans un réacteur d'éthérification opérant à 70 °C et à 20 bars à un débit de 580 kmol/h avec une charge d'éthanol contenant 0,4 % en eau à un débit de 113 kmol/hr. La teneur en eau de cette charge est due au lavage à l'eau réalisé en vue d'obtenir une charge hydrocarbonée comprenant moins de 0,5 ppm d'acétonitrile avant son introduction dans le réacteur. Le flux issu du réacteur est divisé en deux effluents, le premier représentant 60 % du flux est recyclé vers le premier réacteur à un débit de 882 kmol/hr afin d'optimiser le taux de conversion des iso-oléfines. Le second est distillé dans une colonne pour obtenir en fond de colonne l'éther (ETBE) exempt d'hydrocarbures et d'alcool.

En mettant en oeuvre un lavage à l'eau de la charge hydrocarbonée, on obtient 76,4 % mol d'ETBE en fond de colonne de distillation.

### Exemple 3 (selon l'invention)

Au lieu d'effectuer un lavage de la charge hydrocarbonée à l'eau, l'élimination de l'acétonitrile est réalisée en utilisant un liquide ionique. La colonne de séparation liquide-liquide est dimensionnée avec un nombre suffisant de plateaux permettant d'obtenir, après lavage, une charge hydrocarbonée comprenant moins de 0,5 ppm d'acétonitrile.

Le fait de ne plus introduire d'eau dans la charge hydrocarbonée avant l'introduction de ladite charge dans la section réactionnelle permet un gain d'environ 2 % sur la pureté de l'ETBE produit en fond de colonne de séparation. En reprenant les conditions de l'Exemple 2, on obtient 78,2 % mol d'ETBE en fond de colonne de distillation.

## Revendications

1. Procédé de production d'éthers à partir d'une coupe oléfnique contenant au moins une iso-oléfine et d'un alcool, comportant, avant éthérification, une étape a) d'élimination de l'acétonitrile présent dans une charge hydrocarbonée, par extraction liquide-liquide, réalisée dans au moins une zone d'extraction, **caractérisé en ce que** le solvant d'extraction est un liquide ionique non aqueux de formule générale Q⁺A⁻, dans laquelle le cation Q⁺ dérivé d'hétérocycle azoté comportant 1 ou 2 d'azote, l'hétérocycle étant constitué de 5 à 6 atomes, de formules générales 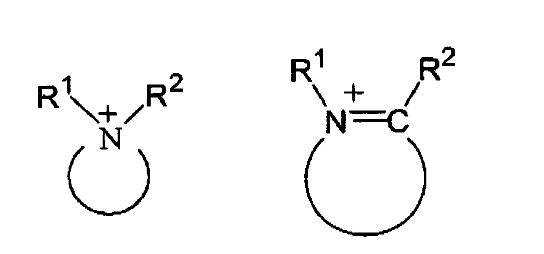 dans lesquelles R¹ et R² , identiques ou différents, représentent l'hydrogéne, de préférence un seul substituant représentant l'hydrogène, ou des radicaux hydrocarbyles ayant de 1 à 30 atomes de carbone
et A⁻ un anion formant avec ledit cation un sel liquide.

2. Procédé selon la revendication 1 **caractérisé en ce que** le mélange soutiré de la zone d'extraction est constitué du liquide ionique chargé en acétonitrile.

3. Procédé selon l'une des revendications 1 à 2 **caractérisé en ce que** le mélange soutiré de la zone d'extraction est envoyé vers une section de régénération du liquide ionique, ledit liquide étant recyclé pour l'extraction.

4. Procédé selon l'une des revendications 1 à 3 **caractérisé en ce que** la régénération du liquide ionique se fait par distillation et/ou détente.

5. Procédé selon l'une des revendications 1 à 4, **caractérisé en ce que** la circulation des liquides dans la colonne de séparation se fait à contre-courant.

6. Procédé selon l'une des revendications 1 à 5 **caractérisé en ce que** l'anion A⁻ est choisi dans les groupes comportant anions halogénures, nitrate, sulfate, alkylsulfates, phosphate, alkylphosphates, acétate, halogénoacétates, tétrafluoroborate, tétrachloroborate, hexafluorophosphate, trifluoro-tris-(pentafluoroethyl)phosphate, hexafluoroantimonate, fluorosulfonate, alkylsulfonates, perfluoroalkylsulfonates, bis(perfluoroalkylsulfonyl)amidures, le méthylure de tris-trifluorométhylsulfonyle de formule C(CF₃SO₂)₃⁻, le méthylure de bis-trifluorométhylsulfonyle de formule HC(CF₃SO₂)₃⁻, arènesulfonates, éventuellement substitués par des groupements halogènes ou halogénoalkyles, l'anion tétraphénylborate et les anions tétraphénylborates dont les noyaux aromatiques sont substitués, le tétra-(trifluoroacétoxy)-borate, le bis-(oxalato)-borate, le dicyanamide et le tricyanométhylure.

7. Procédé selon l'une des revendications précédentes **caractérisé en ce que** les groupes R¹ et R² représentent des groupements alkyles, saturés ou non saturés, cycloalkyles ou aromatiques, aryles ou aralkyles, éventuellement substitués, comprenant de 1 à 30 atomes de carbone

8. Procédé selon l'une des revendications précédentes **caractérisé en ce que** au moins un des groupes R¹ et R² portent une ou plusieurs fonctions choisies parmi les fonctions -CO₂R, -C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR') dans lesquelles R, R' et R", identiques ou différents, représentent chacun l'hydrogène ou des radicaux hydrocarbyles ayant de 1 à 30 atomes de carbone.

9. Procédé selon l'une des revendications précédentes **caractérisé en ce que** le cation est choisi dans le groupe comprenant le N-butylpyridinium, le N-éthylpyridinium, le pyridinium, l'éthyl-3-méthyl-1-imidazolium, le butyl-3-méthyl-1-imidazolium, l'hexyl-3-méthyl-1-imidazdlium, le butyl-3-diméthyl-1,2-imidazolium, le cation (hydroxy-2-éthyl)-1-méthyl-3-imidazolium, le cation (carboxy-2-éthyl)-1-méthyl-3-imidazolium, le diéthylpyrazolium et le N-butyl-N-méthylpyrrolidinium.

10. Procédé selon l'une des revendications précédentes **caractérisé en ce que** le liquide ionique Q⁺A⁻ est choisi dans le groupe comprenant l'hexafluorophosphate de N-butylpyridinium, le bis(trifluorométhylsulfonyl) amidure de butyl-3-méthyl-1-imidazolium, le bis(trifluorométhylsulfonyl) amidure de butyl-3-diméthyl-1,2-imidazolium, le bis(trifluorométhylsulfonyl) amidure de N-butyl-N-méthylpyrrolidinium, le tétrafluoroborate de butyl-3-méthyl-1-imidazolium, le tétrafluoroborate de butyl-3-diméthyl-1,2-imidazolium, le tétrafluoroborate d'éthyl-3-méthyl-1-imidazolium, l'hexafluoroantimonate de butyl-3-méthyl-1-imidazolium, le trifluoroacétate de butyl-3-méthyl-1-imidazolium, le triflate de éthyl-3-méthyl-1-imidazolium, le bis(trifluorométhylsulfonyl)amidure de (hydroxy-2-éthyl)-1-méthyl-3-imidazolium, et le bis(trifluorométhylsulfonyl)amidure de (carboxy-2-éthyl)-1-méthyl-3-imidazolium.

11. Procédé selon l'une des revendications précédentes comportant en outre après l'étape a) les étapes suivantes :
b) mélange de la fraction hydrocarbonée obtenue après l'étape a) avec au moins un flux d'alcool,
c) réaction d'éthérification par réaction du mélange obtenu à l'étape b) en présence d'une résine échangeuse d'ions de manière à obtenir un effluent éther-hydrocarbures-alcool,
d) séparation dans une colonne de fractionnement dudit effluent éther-hydrocarbures-alcool en un premier effluent enrichi en éther et contenant une partie de l'alcool excédentaire et en un deuxième effluent enrichi en hydrocarbures contenant l'autre partie de l'alcool excédentaire.

12. Procédé selon l'une des revendications précédentes **caractérisé en ce que** la coupe oléfinique est issue d'une unité de craquage catalytique.

13. Procédé selon l'une des revendications précédentes **caractérisé en ce que** la fraction hydrocarbonée comprend moins de 50% poids d'iso-oléfirtes.

14. Procédé selon l'une des revendications précédentes **caractérisé en ce que** l'alcool est le méthanol ou l'éthanol.

## Patentansprüche

1. Verfahren zur Herstellung von Ethern aus einem Olefinschnitt, der mindestens ein Isolefin und einen Alkohol enthält, umfassend, vor der Veretherung, einen Schritt a) zur Entfernung des in einer Kohlenwasserstoffbeschickung vorhandenen Acetonitrils durch Flüssigflüssig-Extraktion, die in mindestens einer Extraktionszone ausgeführt wird, **dadurch gekennzeichnet, dass** das Extraktionslösemittel eine nicht wässrige, ionische Flüssigkeit mit der allgemeinen Formel Q⁺A⁻ ist, worin Q⁺ von einem stickstoffhaltigen Heterozyklus abgeleitet ist, der 1 oder 2 Stickstoffatome umfasst, wobei der Heterozyklus aus 5 bis 6 Atomen mit den folgenden allgemeinen Formeln besteht: 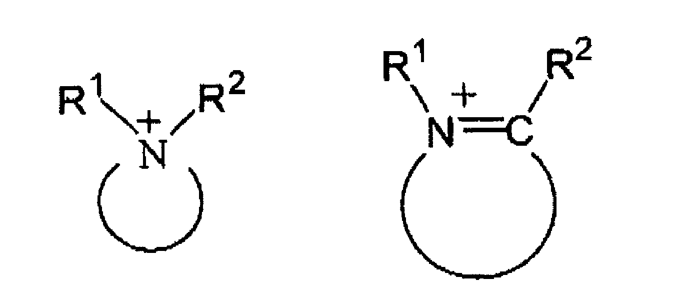 worin R¹ und R², gleich oder verschieden, für Wasserstoff, wobei bevorzugt ein einziger Substituent für Wasserstoff steht, oder Kohlenwasserstoffreste mit 1 bis 30 Kohlenstoffatomen stehen,
und A⁻ für ein Anion steht, das mit dem Kation ein flüssiges Salz bildet.

2. Verfahren nach Anspruch 1 **dadurch gekennzeichnet, dass** das aus der Extraktionszone abgezogene Gemisch aus mit Acetonitril beladener ionischer Flüssigkeit besteht.

3. Verfahren nach einem der Ansprüche 1 bis 2, **dadurch gekennzeichnet, dass** das aus der Extraktionszone abgezogene Gemisch in einen Abschnitt zur Regeneration der ionischen Flüssigkeit geschickt wird, wobei die Flüssigkeit zur Extraktion zurückgeführt wird.

4. Verfahren nach einem der Ansprüche 1 bis 3, **dadurch gekennzeichnet, dass** die Regeneration der ionischen Flüssigkeit durch Destillation und/oder Entspannung erfolgt.

5. Verfahren nach einem der Ansprüche 1 bis 4, **dadurch gekennzeichnet, dass** die Zirkulation der Flüssigkeiten in der Trennsäule mittels Gegenstrom erfolgt.

6. Verfahren nach einem der Ansprüche 1 bis 5, **dadurch gekennzeichnet, dass** das Anion A⁻ ausgewählt ist aus den Gruppen, die folgende Anionen umfassen: Halogenid, Nitrat, Sulfat, Alkylsulfate, Phosphat, Alkylphosphate, Acetat, Halogenoacetate, Tetrafluorborat, Tetrachlorborat, Hexafluorphosphat, Trifluor-tris(pentafluorethyl)phosphat, Hexafluorantimonat, Fluorsulfonat, Alkylsulfonate, Perfluoralkylsulfonate, Bis(perfluoralkylsulfonyl)amide, Tris-trifluormethylsulfonylmethylid mit der Formel C(CF₃SO₂)₃⁻ , Bis-trifluormethylsulfonylmethylid mit der Formel HC(CF₃SO₂)₃⁻, Arensulfonate, die gegebenenfalls mit Halogen- oder Halogenalkylgruppen substituiert sind, dem Tetraphenylboratantion und den Tetraphenylboratanionen, deren aromatischen Kerne substituiert sind, Tetra(trifluoracetoxy)borat, Bis-(oxalato)borat, Dicyanamid und Tricyanamethylid.

7. Verfahren nach einem der vorhergehenden Ansprüche, **dadurch gekennzeichnet, dass** die Gruppen R¹ und R² für gesättigte oder ungesättigte Alkylgruppen, Cycloalkylgruppen oder aromatische Gruppen, Aryl- oder Aralkylgruppen stehen, die gegebenenfalls substituiert sind und 1 bis 30 Kohlenstoffatome umfassen.

8. Verfahren nach einem der vorhergehenden Ansprüche, **dadurch gekennzeichnet, dass** mindestens eine der Gruppen R¹ und R² eine oder mehrere Funktionen trägt, ausgewählt aus den Funktionen -CO₂R, - C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), - P(O)(OR)(OR'), worin R, R' und R", gleich oder verschieden, jeweils für Wasserstoff oder Kohlenwasserstoffreste mit 1 bis 30 Kohlenstoffatomen stehen.

9. Verfahren nach einem der vorhergehenden Ansprüche, **dadurch gekennzeichnet, dass** das Kation ausgewählt ist aus der Gruppe, umfassend N-Butylpyridinium, N-Ethylpyridinium, Pyridinium, Ethyl-3-methyl-1-imidazolium, Butyl-3-methyl-1-imidazolium, Hexyl-3-methyl-1-imidazolium, Butyl-3-dimethyl-1,2-imidazolium, (Hydroxy-2-ethyl)-1-methyl-3-imidazoliumkation, (Carboxy-2-ethyl)-1-methyl-3-imidazoliumkation, Diethylpyrazolium und N-Butyl-N-methylpyrrolidinium.

10. Verfahren nach einem der vorhergehenden Ansprüche, **dadurch gekennzeichnet, dass** die ionische Flüssigkeit Q⁺A⁻ ausgewählt ist aus der Gruppe umfassend N-Butylpyridiniumhexafluorphosphat, Butyl-3-methyl-1-imidazolium-bis(trifluormethylsulfonyl)amid, Butyl-3-dimethyl-1,2-imidazolium-bis(trifluormethylsulfonyl)amid, N-Butyl-N-methylpyrrolidinium-bis(trifluormethylsulfonyl)amid, Butyl-3-methyl-1-imidazolium-tetrafluorborat, Butyl-3-dimethyl-1,2-imidazolium-tetrafluorborat, Ethyl-3-methyl-1-imidazolium-tetrafluorborat, Butyl-3-methyl-1-imidazolium-hexafluorantimonat, Butyl-3-methyl-1-imidazolium-trifluoracetat, Ethyl-3-methyl-1-imidazolium-triflat, (Hydroxy-2-ethyl)-1-methyl-3-imidazolium-bis(trifluormethylsulfonyl)amid, (Carboxy-2-ethyl)-1-methyl-3-imidazolium-bis(trifluormethylsulfonyl)amid.

11. Verfahren nach einem der vorhergehenden Ansprüche, das nach Schritt
a) ferner die folgenden Schritte umfasst:
b) Mischen der nach Schritt a) erhaltenen Kohlenwasserstofffraktion mit mindestens einem Alkoholstrom,
c) Veretherungsreaktion durch Umsetzen des in Schritt b) erhaltenen Gemischs in Gegenwart eines Ionenaustauscherharzes, um einen Ether-Kohlenwasserstoffe-Alkohol-Abfluss zu erhalten,
d) Trennen des Ether-Kohlenwasserstoffe-Alkohol-Abflusses in einer Fraktionierungssäule in einen ersten Abfluss, der mit Ether angereichert ist und einen Teil des überschüssigen Alkohols enthält, und in einen zweiten Abfluss, der mit Kohlenwasserstoffen angereichert ist und den anderen Teil des überschüssigen Alkohols enthält.

12. Verfahren nach einem der vorhergehenden Ansprüche, **dadurch gekennzeichnet, dass** der Olefinschnitt aus einer Einheit zum katalytischen Cracken stammt.

13. Verfahren nach einem der vorhergehenden Ansprüche, **dadurch gekennzeichnet, dass** die Kohlenwasserstofffraktion mindestens 50 Gew.-% Isoolefine umfasst.

14. Verfahren nach einem der vorhergehenden Ansprüche, **dadurch gekennzeichnet, dass** der Alkohol Methanol oder Ethanol ist.

## Claims

1. A process for producing ethers from an olefinic cut containing at least one isoolefin and from an alcohol comprising, prior to etherification, a stage a) of removal of the acetonitrile present in a hydrocarbon feed by liquid-liquid extraction, performed in at least one extraction zone, **characterized in that** the extraction solvent is a non-aqueous ionic liquid of general formula Q⁺A⁻, wherein Q⁺ wherein Q⁺ is derived from a nitrogen-containing heterocycle comprising 1 or 2 nitrogen atoms, of general formulas: 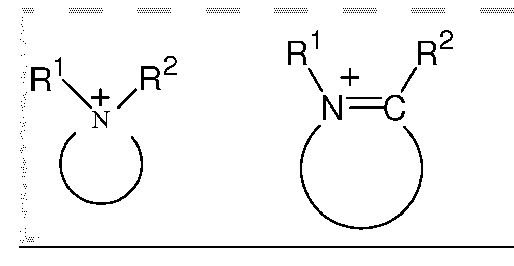
wherein the heterocycle consists of 5 to 6 atoms, and R¹ and R², identical or different, represent each hydrogen, preferably a single substituent representing hydrogen, or hydrocarbyl radicals having 1 to 30 carbon atoms.
and A⁻ an anion forming a liquid salt with said cation.

2. A process as claimed in claim 1, **characterized in that** the mixture discharged from the extraction zone consists of the acetonitrile-laden ionic liquid.

3. A process as claimed in any one of claims 1 and 2, **characterized in that** the mixture discharged from the extraction zone is sent to an ionic liquid regeneration section, said liquid being recycled for extraction.

4. A process as claimed in any one of claims 1 to 3, **characterized in that** regeneration of the ionic liquid is performed by distillation and/or expansion.

5. A process as claimed in any one of claims 1 to 4, **characterized in that** circulation of the liquids in the separation column takes place in a counter-current flow.

6. A process as claimed in any one of claims 1 to 5, **characterized in that** the A⁻anion is preferably selected from among groups comprising the following anions: halogenides, nitrate, sulfate, alkylsulfates, phosphate, alkylphosphates, acetate, halogenoacetates, tetrafluoroborate, tetrachloroborate, hexafluorophosphate, trifluoro-tris-(pentafluoroethyl)phosphate, hexafluoroantimonate, fluorosulfonate, alkyl-sulfonates, perfluoroalkylsulfonates, bis(perfluoroalkylsulfonyl)amidides, tris-trifluoromethylsulfonyl methylide of formula C(CF₃SO₂)₃⁻, bis-trifluoromethylsulfonyl methylide of formula HC(CF₃SO₂)₃⁻, arenesulfonates, possibly substituted by halogen or halogeno-alkyl groups, the tetraphenylborate anion and the tetraphenylborate anions whose aromatic rings are substituted, tetra(trifluoroacetoxy)borate, bis(oxalato)borate, dicyanamide and tricyanomethylide.

7. A process as claimed in any one of claims 1 to 6, **characterized in that** the groups R¹ and R² represent alkyl groups, saturated or non-saturated, cycloalkyls or aromatics, aryls or aralkyls, possibly substituted, comprising 1 to 30 carbon atoms.

8. A process as claimed in any one of the previous claims, **characterized in that** at least one of groups R¹ and R², carries one or more functions selected from among the following functions: -CO₂R, -C(O)R, -OR, -C(O)NRR', -C(O)N(R)NR'R", -NRR', -SR, -S(O)R, -S(O)₂R, -SO₃R, -CN, -N(R)P(O)R'R', -PRR', -P(O)RR', -P(OR)(OR'), -P(O)(OR)(OR'), wherein R, R' and R", identical or different, represent each hydrogen or hydrocarbyl radicals having 1 to 30 carbon atoms.

9. A process as claimed in any one of the previous claims, **characterized in that** the cation is selected from the group comprising: N-butylpyridinium, N-ethylpyridinium, pyridinium, ethyl-3-methyl-1-imidazolium, butyl-3-methyl-1-imidazolium, hexyl-3-methyl-1-imidazolium, butyl-3-dimethyl-1,2-imidazolium, the (hydroxy-2-ethyl)-1-methyl-3-imidazolium cation, the (carboxy-2-ethyl)-1-methyl-3-imidazolium cation, diethylpyrazolium, N-butyl-N-methylpyrrolidinium.

10. A process as claimed in any one of the previous claims, **characterized in that** the Q⁺ A⁻ ionic liquid is selected from the group comprising: N-butylpyridinium hexafluorophosphate" butyl-3-methyl-1-imidazolium bis(trifluoro-methylsulfonyl)amidide, butyl-3-dimethyl-1,2-imidazolium bis(trifluoro-methylsulfonyl)amidide, N-butyl-N-methylpyrrolidinium bis(trifluoro-methylsulfonyl)amidide, butyl-3-methyl-1-imidazolium tetrafluoroborate, butyl-3-dimethyl-1,2-imidazolium tetrafluoroborate, ethyl-3-methyl-1-imidazolium tetrafluoroborate, butyl-3-methyl-1-imidazolium hexafluoroantimonate, butyl-3-methyl-1-imidazolium trifluoroacetate, ethyl-3-methyl-1-imidazolium triflate, (hydroxy-2-ethyl)-1-methyl-3-imidazolium bis(trifluoromethylsulfonyl)amidide, (carboxy-2-ethyl)-1-methyl-3-imidazolium bis(trifluoromethylsulfonyl)amidide.

11. A process as claimed in any one of the previous claims, further comprising, after stage a), the following stages:
b) mixing the hydrocarbon fraction obtained after stage a) with at least one alcohol stream,
c) etherification reaction by reaction of the mixture obtained in stage b) in the presence of an ion exchange resin so as to obtain an ether-hydrocarbon-alcohol effluent,
d) separating in a fractionation column said ether-hydrocarbon-alcohol effluent into a first effluent enriched in ether and containing part of the excess alcohol and into a second effluent enriched in hydrocarbons containing the other part of the excess alcohol.

12. A process as claimed in any one of the previous claims, **characterized in that** the olefinic cut comes from a catalytic cracking unit.

13. A process as claimed in any one of the previous claims, **characterized in that** the hydrocarbon fraction comprises less than 50 wt.% iso-olefins.

14. A process as claimed in any one of the previous claims, **characterized in that** the alcohol is methanol or ethanol.
